Europäisches Patentamt

European Patent Office

Office européen des brevets

(11) Publication number: 0 504 947 A2

## EUROPEAN PATENT APPLICATION

(21) Application number: 92105010.0

(22) Date of filing: 23.03.92

(51) Int. Cl.5: **C12N 9/24**, C12N 1/14,
//(C12N9/24,C12R1:785)

(30) Priority: **22.03.91 YU 531/91**

(43) Date of publication of application:
**23.09.92 Bulletin 92/39**

(84) Designated Contracting States:
**AT DE DK NL**

(71) Applicant: **KRKA, tovarna zdravil, p.o.**
**Cesta herojev 45**
**YU-68000 Novo Mesto(YU)**

(72) Inventor: **Filipovic, Branislav**
**Cesta herojev 30**
**Y-68000 Novo mesto(YU)**
Inventor: **Pokorny, Miroslav, Dr.-Ing.**
**Cankarjeva 11**
**Y-68000 Novo mesto(YU)**

(74) Representative: **Deufel, Paul, Dr. et al**
**Müller-Boré & Partner Patentanwälte**
**Isartorplatz 6 Postfach 26 02 47**
**W-8000 München 2(DE)**

(54) **Process for the production of beta glucanase from the mould Mucor miehei and beta glucanase produced by this process.**

(57) There is disclosed a process for the preparation of a beta glucanase from the mould *Mucor miehei*, where the microorganism *Mucor miehei* MZKIBK B-196 is cultivated under submersed aerobic conditions at a pH from 5.7 to 6.4 and at a temperature from 28 to 36°C at first in the vegetative phase and subsequently in the vegetative phase in a nutrient culture medium consisting of a carbon source, a nitrogen source, of vitamins and mineral substances.

*Technical Field*

The invention belongs to the field of microbiology and relates to a process for the production of beta glucanase from the mould *Mucor miehei*, to beta glucanase produced by that process as well as to the use of that enzyme as an enzyme preparation. The mould *Mucor (Rhizomucor) miehei* is deposited in the microbiological collection of the Kemijski inštitut Boris Kidrič in Ljubljana under the No. MZKIBK B-196.

*Technical Problem*

There existed a need for developing an economical and quality industrial process for the production of beta glucanase.

*Prior Art*

Beta glucanases are hydrolytic glycosidasic enzymes cleaving the $\beta$-1,3-bond in different polysaccharides to glucose and oligosaccaharides. According to the international nomenclature for enzymes, such an enzyme has the reference E.C.3.2.1.6. Polysaccharide substances containing the $\beta$-1,3-bond are beta glucanes, which are present in greater amounts in cereal seeds (barley 1-2%, wheat 0.5-10%, maize under 0.2%, oat under 1.0%), partly in some kinds of algae (laminarin), in lichens (lichenan) and moulds (biotricidin). In cells, beta glucanes have a mechanical function (a constituent part of membranes) or represent reserve carbohydrates. These compounds often contain some $\beta$-1,4- and $\beta$-1,6-bonds and because of such bonds the molecules are branched and cross-linked. Such compounds have a great affinity to water and in water solutions they form stable collodial slimy solutions. For animals and for people, beta glucanes are without any nutritive value because their digestive system does not contain beta glucanase to decompose beta glucanes.

Beta glucanes and beta glucanases became interesting no more than a few years ago, due the intensity and modernization of the food industry (production of beer, juices, wine, alcohol), breeding of animals and human dietary nutrition.

Well known are beta glucanases arising from different malts (barley, wheat), where they are found as a mixture with different hydrolytic enzymes (amylases, proteases). The industrial production of beta glucanases from malts is complicated and expensive. Therefore different microorganisms have been examined as potential producers of beta glucanases. The results of this research have made possible the industrial production by means of microorganisms. In practice the following microorganisms-producers are used: *Penicillinum emersonic*, *Bacillus amyloliquefaciens*, *Bacillus subtilis*, *Aspergillus niger* and *Trichoderma reesei*. In the literature different streptomycetes and basidiomycetes are sometimes mentioned as potential producers, however, no economical and quality industrial processes have been developed so far.

*Technical Solution*

Our research in the field of biosynthesis of microbiological rennet by means of *Mucor (Rhizomucor) miehei* have shown that single isolates of this mould demonstrate the ability of biosynthesis of inducible extracellular beta glucanase. For the induction of beta glucanase, glucanes from barley, oat, wheat and their malts were used. For that purpose, a selection of more potent strains was carried out and then the culture medium was adapted and optimized.

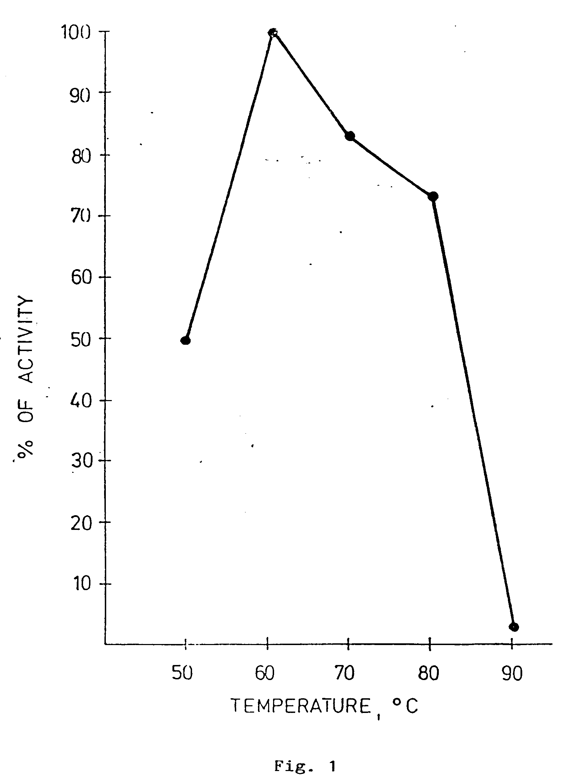

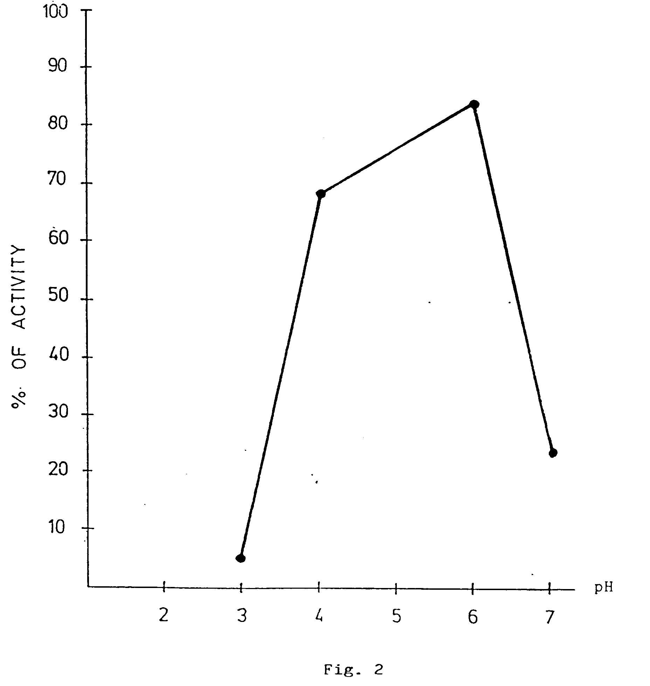

For the detection of enzyme activity, there was used the decomposition of substrate lichenan up to reductive sugars, which were detected by p-nitrosalicylic acid. This is shown in Fig. 1 and Fig.2. In Fig. 1 there is represented a graph of the dependence of the enzyme activity upon the hydrolysis temperature of lichenan. In Fig.2 there is represented a graph of the dependence of the enzyme activity upon the pH at the hydrolysis of lichenan.

On the basis of these statements the fermentation substrate was composed, containing a source of carbon (e.g. vegetable meal containing beta glucanes), a source of nitrogen (e.g. soya meal, brewer's yeast, $(NH_4)_2SO_4$) and anorganic salts ($MnSO_4$, $CoCl_2$, $ZnSO_4$, $MgSO_4$, $FeSO_4$, $NaH_2PO_4$ and $KH_2PO_4$).

Thus, the process for the production of beta glucanase from mould *Mucor miehei* according to this invention is carried out so that the microorganism *Mucor miehei* MZKIBK B-196 is cultivated under submersed aerobic conditions at a pH of 5.7 to 6.4, preferably 5.7 to 6.2, and at temperature from 28 to 36°C first in the vegetative phase and subsequently in the fermentation phase in a nutrient culture medium consisting of a carbon source, a nitrogen source, of vitamins and mineral materials.

By such a composition of the substrate a greater biosynthesis of extracellular beta glucanase is achieved, on the laboratory scale even 270 beta glucanase units (BGu) per ml of the culture filtrate of the age of 120-150 hours. Such a yield makes possible a direct use of this filtrate as a commercial preparation. Standard commercial preparations have contents from 150 BGu/ml on.

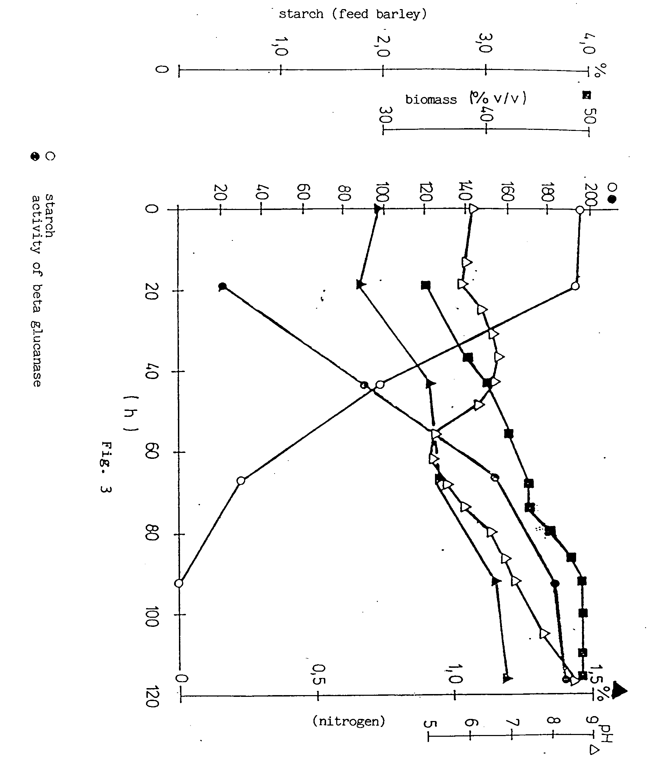

In any case the content of enzyme depends upon the type and concentration of the inductor substrate and upon the conditions of biosynthesis as it is shown in Fig. 3, where the time course of beta glucanase biosynthesis in a 1500 l fermentor at 36°C, an aeration rate of 0.6 v/v/min and mixing rate of 200 rpm is illustrated.

The most intensive biosynthesis of beta glucanase takes place in the phase of morphological modification of the producer hifae to the pellet form.

After the completion of the fermentation, the broth is cooled down and the biomass is separated from the filtrate containing the enzyme. Stabilizers (KCl, $CaCl_2$ or $MgCl_2$) and preservatives like NaCl and sodium benzoate are added to the filtrate and then it is used as a commercial preparation or it is concentrated and purified by ultrafiltration. As the molecular weight of enzyme is 30000 and 40000 daltons, respectively, membranes determining the molecular weight of about 30000 daltons are used.

In the method of ultrafiltration, 90 to 100% yields of activity are achieved, depending on the degree of concentration. Sodium benzoate and the stabilizer $CaCl_2$ are added to the concentrated preparation and its activity is standardized in view of the commercial activity. The preparation prepared in such a way is stable for more than 6 months at room temperature.

The concentrated ultrafiltrate can be dried on a spray drier or the enzyme is isolated by anorganic salts or solvents and thus a strongly concentrated preparation is obtained (from 1000 to 2000 BGu/g).

The beta glucanase preparation (liquid or powdery) has an interesting composition. In addition to beta glucanase it contains some alpha amylase and some neutral protease. It does not have any cellulase or hemicellulase activity, which is very interesting because the commercial preparations of beta glucanases obtained from *Trichodermae* or *Aspergilli* contain a considerable amount of cellulase and hemicellulase components. The preparation of beta glucanase is active in a wide pH range, namely from 3 to 7, but the optimum pH is about 4 to 6. The optimum working temperature is between 60 and 70°C, which makes this enzyme one of the so-called thermostable beta glucanases with the optimum temperature below 62°C.

Such enzyme preparations are very effective in:

a) beer production, namely in the phase of preparing the wort (mashing) and in the fermentation phase. In both phases, sufficient amounts of betaglucanes from barley, maize or wheat or their malts are decomposed;

b) clarification of fruit juices and wines, whereby they partially or completely decompose the soluble glucanes from fruits or existing microorganisms;

c) composition of enzyme cocktails for animal food (pig breeding, fish, cattle).

The invention is illustrated by the following Examples:

*Example 1*

The microorganism *Mucor* (*Rhizomucor*) *miehei* K-BGL-01 (MZKIBK B-196) was cultivated on the nutrient agar consisting components, as follows:

| | |
|---|---|
| lactose | 10 g |
| brewer's yeast | 20 g |
| $(NH_4)_2HPO_4$ | 2.5 g |
| casein | 2.5 g |
| agar | 20 g |
| demineralized water *ad* 1000 ml | |

The culture medium was previously sterilized at 121°C for 30 minutes. To the culture medium thus prepared, a suspension of spores *M. miehei* was added and it was incubated at 32°C for 10 to 15 days. The sporulated mycelium which overgrew the surface of the nutrient aggregate was resuspended in 25 ml of sterile distilled water. The suspension of spores thus prepared was added, in an amount of 2.0 vol.% to the sterile vegetative culture medium of the following composition:

| brewing malt (meal) | 15 g |
| soya (meal) | 20 g |
| dry alcohol yeast | 1.2 g |
| $MnSO_4.H_2O$ | 0.005 g |
| $CoCl_2.6H_2O$ | 0.0005 g |
| $ZnSO_4.7H_2O$ | 0.05 g |
| $MgSO_4.7H_2O$ | 0.001 g |
| $FeSO_4.7H_2O$ | 0.0125g |
| antifoaming silicone 1510 (Dow Corning) | 6.6 ml |
| tap water *ad* 1000 ml | |

The culture medium was previously boiled and then cooled to the temperature of 22°C (±2°C) and the pH was corrected by 50% lactic acid to 5.55.

It was sterilized at 121°C for 30 minutes. The pH after sterilisation was 5.9 to 6.2.

The vegetative phase of the microorganism growth was 25 to 48 hours, preferably 48 hours at a temperature from 28 to 36°C and at 220 to 250 rpm, preferably at 220 rpm, and was used as inoculum; the pH of the inoculum was 7.4 to 8.3.

The fermentation culture medium was inoculated with 3 to 5 vol.% of the vegetative culture, preferably with 4 vol.%. The composition of the fermentation culture medium was as follows:

| barley meal (brewer's) | 7.2% |
| soya meal | 3.0% |
| dry brewer's yeast | 0.18% |
| $(NH_4)_2SO_4$ | 1.5% |
| $NaH_2PO_4$ | 0.15% |
| antifoamimg silicon 1510 (Dow Corning) | 0.385% |
| tap water *ad* 1000 ml | |

The culture medium was sterilized at 121°C for 90 minutes, the pH after sterilization was 5.9 to 6.4. After the fermentation was completed in 120 to 150 hours at 36°C and at 250 rpm in the rotary shaker, the content of beta glucanase in the broth or in the filtrate of the broth, respectively, was 220 u/ml.

*Example 2*

The broth was filtered by means of a filtering agent, $CaCl_2$, NaCl, KCl and sodium benzoate were added to the filtrate containing 200 BGu/ml and it was used as a commercial preparation.

*Example 3*

It was proceeded as in Example 1 with the exception that the fermentation culture medium contained feed barley instead of brewer's quality barley. After the fermentation was completed in 120 to 150 hours, the content of beta glucanase in the broth or in the filtrate of the broth, respectively, was 250 u/ml.

The filtrate of the broth was cooled to 10 to 15°C and then concentrated to a 1/4 of volume on a ultrafiltration device (Millipore) separating molecules with molecular weight of 10000. To the concentrate containing 950 BGu/ml (95% yield), the same fillers as in Example 1 was added and it was used as commercial preparation.

*Example 3*

It was proceeded as in Example 1 with the exception that the fermentation culture medium additionaly contained:

| MnSO$_4$.H$_2$O | 0.005 g |
|---|---|
| CoCl$_2$.6H$_2$O | 0.0005 g |
| ZnSO$_4$.7H$_2$O | 0.05 g |
| MgSO$_4$.7H$_2$O | 0.001 g |
| FeSO$_4$.7H$_2$O | 0.0125 g |

After the fermentation was completed (after 120 to 150 hours), the content of beta glucanase in the broth and in the filtrate of the broth, respectively, was 280 u/ml.

**Claims**

1. A process for the production of beta glucanase from the mould *Mucor miehei*, **characterized in that** the microorganism *Mucor miehei* MZKIBK B-196 is cultivated under submersed aerobic conditions at a pH from 5.7 to 6.4 and a temperature from 28 to 36°C at first in the vegetative phase and subsequently in the fermentation phase in a nutrient culture medium consisting of a carbon source, a nitrogen source, of vitamins and mineral substances.

2. A process according to claim 1, characterized in that as the carbon source there are used vegetable meals containing natural beta glucanases, preferably barley, maize and wheat meals as well as the meal of malts thereof.

3. A process according to claim 1, characterized in that as the nitrogen source soya meal and dry brewer's yeast are used as organic nitrogen sources and (NH$_4$)$_2$SO$_4$ is used as an anorganic nitrogen source.

4. A process according to claim 1, characterized in that as mineral salts there are used MnSO$_4$.H$_2$O, CoCl$_2$.6H$_2$O, ZnSO$_4$.7H$_2$O, MgSO$_4$.7H$_2$O, FeSO$_4$.7H$_2$O and NaH$_2$PO$_4$ or KH$_2$PO$_4$.

5. Beta glucanase as produced by the process according to claim 1.

6. A beta glucanase preparation, characterized in that it comprises the broth filtrate after complete fermentation according to claim 1 as well as stabilizers such as MgCl$_2$, CaCl$_2$ and KCl in liquid, powdery or granular form.

Fig. 1

Fig. 2

Fig. 3